(19) Europäisches Patentamt / European Patent Office / Office européen des brevets

(11) **EP 3 297 716 B1**

(12) **FASCICULE DE BREVET EUROPEEN**

(45) Date de publication et mention de la délivrance du brevet:
**22.04.2020 Bulletin 2020/17**

(21) Numéro de dépôt: **16725096.8**

(22) Date de dépôt: **20.05.2016**

(51) Int Cl.:
***A61M 25/00*** *(2006.01)*

(86) Numéro de dépôt international:
**PCT/EP2016/061409**

(87) Numéro de publication internationale:
**WO 2016/188894 (01.12.2016 Gazette 2016/48)**

(54) **CATHETER VASCULAIRE PERMETTANT L'INJECTION D'UN VOLUME TYPE BOUCHON**

GEFÄSSKATHETER ZUR INJEKTION EINES PFROPFARTIGEN VOLUMENS

VASCULAR CATHETER PERMITTING THE INJECTION OF A VOLUME OF THE PLUG TYPE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.05.2015 FR 1554638**

(43) Date de publication de la demande:
**28.03.2018 Bulletin 2018/13**

(73) Titulaires:
• **Université Paris Diderot - Paris 7**
**75013 Paris (FR)**
• **Centre National de la Recherche Scientifique (CNRS)**
**75016 Paris (FR)**

(72) Inventeurs:
• **GUIFFANT, Gérard**
**75012 Paris (FR)**
• **MERCKX, Jacques**
**75015 Paris (FR)**
• **FLAUD, Patrice**
**77380 Combs-la-Ville (FR)**

(74) Mandataire: **Regimbeau**
**20, rue de Chazelles**
**75847 Paris Cedex 17 (FR)**

(56) Documents cités:
WO-A1-97/21455          WO-A1-2011/091275
WO-A2-01/51116          US-A- 5 098 413
US-A- 5 536 261          US-A1- 2011 046 600
US-A1- 2011 224 625    US-A1- 2013 053 826

EP 3 297 716 B1

**Description**

DOMAINE DE L'INVENTION

La présente invention concerne le domaine des cathéters vasculaires permettant l'injection d'un volume type bouchon soit pour injection courante soit pour opacification vasculaire. Le volume injecté est fréquemment appelé "bolus" par les équipes soignantes et ces cathéters sont souvent associés à des hautes pressions.

ETAT DE LA TECHNIQUE

L'injection parentérale et plus particulièrement intravasculaire, de produits biologiques, pharmacologique ou chimiques est de pratique courante.

Parmi ces conduites, l'examen par produits de contraste est une technique d'imagerie médicale permettant dans son principe la visualisation d'une structure anatomique (organe creux ou arborescence vasculaire). L'injection du produit de contraste se fait généralement par voie veineuse ou artérielle via un cathéter vasculaire. Quel qu'en soit le type, les cathéters vasculaires sont, pour la plupart à "terminaison ouverte", c'est-à-dire que leur lumière, unique ou multiple, terminale et étagée, s'ouvre à plein canal dans le vaisseau ciblé : artère ou veine. L'injection se fait en une fois à débit important pour favoriser la formation d'un bolus.

Le produit injecté quel que soit le débit, est, plus ou moins rapidement, dilué dans le sang dont le flux moyen est en axillaire 4-5mL/s et en cave de 20 mL/s. L'endoveine est ainsi protégé de l'effet corrosif des cytolytiques, antibiotiques, médicaments divers et produits hyperosmolaires. Pour obtenir un bolus de produit de contraste, compatible avec une opacification vasculaire ou parenchymateuse "exploitable", les débits d'injection utilisés sont de 1 à 5 mL/s. Cette injection produit par réaction, un coup de fouet, à deux composantes horizontale et verticale susceptible de mobiliser l'extrémité du cathéter, responsable de lésion de l'endothélium vasculaire et /ou de migration inappropriée, dans le territoire vasculaire artériel ou veineux.

EXPOSE DE L'INVENTION

La présente invention concerne un cathéter vasculaire mono-lumière destiné à être inséré dans un canal de flux sanguin pour l'injection d'un produit par voie veineuse ou artérielle selon la revendication 1 ainsi que les revendications dépendantes 2-15.

EXPOSE DE LA PRESENTE DESCRIPTION

Le cathéter comporte un tube ayant un corps longitudinal creux présentant une lumière et s'étendant le long de l'axe longitudinal, la lumière s'étendant tout le long du tube et étant destinée à transporter et à injecter un volume donné de produit, selon un débit prescrit.

Conformément à l'invention, le tube présente :

- des orifices latéraux permettant la sortie du volume, de taille sensiblement identique et disposés sensiblement symétriquement par rapport à l'axe longitudinal la lumière dans le corps longitudinal, uniquement à proximité et avant l'extrémité distale du tube selon la direction de l'axe longitudinal (X),
- à partir de l'extrémité distale du tube selon la direction de l'axe longitudinal (X), une surface d'extrémité réduisant totalement ou partiellement une surface totale de la lumière à l'extrémité distale du tube.

Avantageusement, les orifices latéraux et la surface d'extrémité sont agencés et dimensionnés pour que le volume sorte radialement du cathéter principalement par les orifices latéraux et entoure l'extrémité du cathéter, afin de stabiliser le tube et d'injecter le produit hors du cathéter radialement dans l'écoulement du canal de flux sanguin longitudinal au tube.

L'injection de produit hors du tube est essentiellement radiale par rapport au tube au niveau des orifices latéraux, la composante longitudinale de l'injection hors du tube au niveau des orifices latéraux étant négligeable par rapport à la composante radiale, par exemple égale à 10 à 15% de la composante radiale, adaptée à la section du guide.

L'invention concerne les cathéters permettant l'injection de produits de contrastes, mais concerne également les cathéters permettant l'injection d'autres produits à débits plus faibles que ceux utilisés pour les produits de contrastes. Par exemple, elle concerne les cathéters permettant d'une part l'injection de produits (antibiotiques, alimentation parentérale, cytolytiques) et d'autre part, les prélèvements sanguins et les prises de pression cardiovasculaires.

Par ailleurs, aujourd'hui, il peut être utilisé un même cathéter pour faire une première injection d'un produit puis une injection d'un produit de contraste et le cathéter de l'invention permet cela.

En d'autres termes, le cathéter à ouverture latérale distale de l'invention peut être utilisé quelle que soit l'extrémité distale : droite pour une durée ponctuelle (opacification vasculaire) ou tronconique pour des injections successives de produits différents dont le produit de contraste (usage thérapeutique et d'opacification vasculaire).

Il peut être installé de façon provisoire ou prolongée.

La surface d'extrémité présente une forme ayant une diminution progressive de la taille de la lumière, depuis l'extrémité, distale, percée d'un orifice. Une partie extérieure distale du corps longitudinal est située entre les orifices latéraux et la surface d'extrémité du tube, et est entourée par le volume de produit injecté hors du cathéter.

L'invention permet de :

(i) réduire ou supprimer l'effet de fouet,

(ii) améliorer la qualité du bolus en sortie de cathéter en améliorant le remplissage du vaisseau.

En supprimant l'effet de fouet, on améliore la fiabilité de l'examen en supprimant les migrations inappropriées du cathéter.

En améliorant la formation du bolus, on améliore la qualité de l'examen.

L'invention permet ainsi une injection de type bouchon en supprimant ou en limitant tout effet de recul du cathéter dans l'espace vasculaire artériel ou veineux.

Avantageusement, la surface d'extrémité réduit au moins 90% d'une surface totale de la lumière à l'extrémité distale du tube.

Avantageusement, les orifices latéraux et la surface d'extrémité sont agencés et dimensionnés pour qu'au moins 90% du volume sorte du cathéter par les orifices latéraux.

Avantageusement, pour une lumière du cathéter présentant une surface totale donnée et un rayon R donné, les orifices latéraux présentent des dimensions, notamment une dimension longitudinale telle qu'un rayon s'ils sont circulaires, calculée pour qu'une somme des surfaces individuelles des orifices latéraux soit au moins égale à la surface totale donnée de la lumière.

Avantageusement, une dimension longitudinale des orifices latéraux est calculée pour être inférieure au rayon R divisé par $\sqrt{2}$.

Avantageusement, les orifices latéraux présentent une dimension calculée pour que, à longueur égale, la différence entre une résistance hydraulique totale calculée du cathéter, par rapport à une résistance hydraulique totale de référence calculée d'un cathéter à terminaison ouverte destiné à s'ouvrir à plein canal et ayant à l'extrémité un orifice longitudinal de sortie correspondant à la lumière, soit avantageusement inférieure à 5% de la résistance hydraulique de référence.

Avantageusement, le tube comporte deux orifices latéraux distaux en regard.

Avantageusement, pour un usage thérapeutique et radiologique ou cardiologique, la surface d'extrémité distale présente une forme tronconique convergente, dépassant de l'extrémité, percée d'un orifice central.

Avantageusement, la longueur de la partie extérieure distale entre les orifices latéraux et l'extrémité du tube est inférieure au rayon du cathéter.

Avantageusement, les orifices latéraux sont positionnés et présentent des dimensions, notamment une dimension longitudinale telle qu'un rayon s'ils sont circulaires, calculées pour qu'une concentration du volume de produit en sortie de cathéter, selon un débit prescrit, tende à s'homogénéiser sur une distance radiale, par rapport à un axe longitudinal central du cathéter et en sortie de l'extrémité du cathéter, voisine d'un diamètre du canal de flux sanguin.

Avantageusement, les orifices latéraux sont positionnés et présentent des dimensions, notamment une dimension longitudinale telle qu'un rayon s'ils sont circulaires, calculées pour que le volume de produit en sortie de cathéter, selon un débit prescrit, soit réparti sur une distance radiale, avec avantageusement une concentration qui tend à s'homogénéiser sur cette distance radiale, par rapport à un axe longitudinal central du cathéter et en sortie de l'extrémité du cathéter, au moins égale à quatre fois un rayon R de la lumière du tube.

Avantageusement, pour un usage ponctuel, radiologique ou cardiologique, la surface d'extrémité distale ferme complètement la lumière, le volume étant injecté en sortie du cathéter à 100% par les orifices latéraux.

Avantageusement, l'autre extrémité, proximale, du cathéter est apte à être raccordée à un embout connectable type luer.

Avantageusement, les orifices latéraux sont réalisés transversalement au corps longitudinal.

Avantageusement, à titre illustratif et non limitatif, le corps longitudinal est cylindrique de rayon inférieur à 5mm.

Enfin, les orifices latéraux (5) peuvent être dimensionnés pour faire sortir des débits d'injection de bolus de 0,5 à 5 mL/s, à des pressions de l'ordre de 551,58 à 2413,17 KPa (80 à 350 psi).

DESCRIPTION DES FIGURES

D'autres objectifs, caractéristiques et avantages sortiront de la description détaillée qui suit en référence aux dessins donnés à titre illustratif et non limitatif parmi lesquels :

- les figures 1a et 1b représentent respectivement un mode de réalisation selon l'invention et un exemple ne formant pas partie de l'invention ; la figure 1-a représente schématiquement l'extrémité du cathéter comportant deux orifices latéraux symétriques et un orifice de sortie ; la figure 1-b représente schématiquement l'extrémité du cathéter comportant deux orifices latéraux symétriques ;
- les figures 2a et 2b représentent une vue en coupe d'une simulation numérique de l'effet bolus (injection d'un volume donné) à la sortie d'un cathéter comportant deux orifices latéraux symétriques ;
- la figure 3 représente une vue en coupe d'une simulation numérique de l'injection à la sortie d'un cathéter de l'état de l'art comportant un seul orifice distal (à terminaison ouverte);
- la figure 4 représente une vue en coupe de la répartition radiale de concentration à la sortie du cathéter dans les conditions des figures 2a et 2b, selon, respectivement, un mode de réalisation selon l'invention et un exemple ne formant pas partie de l'invention ;
- la figure 5 représente une vue en coupe de la répartition radiale de concentration à la sortie du cathéter dans les conditions de la figure 3, correspondant à une injection classique ;

- la figure 6 représente l'extrémité du cathéter comportant deux orifices latéraux symétriques selon un mode de réalisation;

- la figure 7 représente l'extrémité du cathéter selon respectivement un exemple ne formant pas partie de l'invention et un mode de réalisation selon l'invention, 7a deux orifices latéraux symétriques et une extrémité droite et 7b deux orifices latéraux symétriques et un orifice distal ;

- la figure 8 représente les résistances hydrauliques (rapportées à la résistance hydraulique d'une extrémité classique) pour l'extrémité d'un cathéter dans les différentes situations présentées : ouverture unique axiale, ouvertures latérales symétriques avec ou sans orifice de sortie ;

- la figure 9 représente la résistance hydraulique d'un cathéter de rayon interne 1mm; en fonction de la longueur, dans les différentes situations présentées.

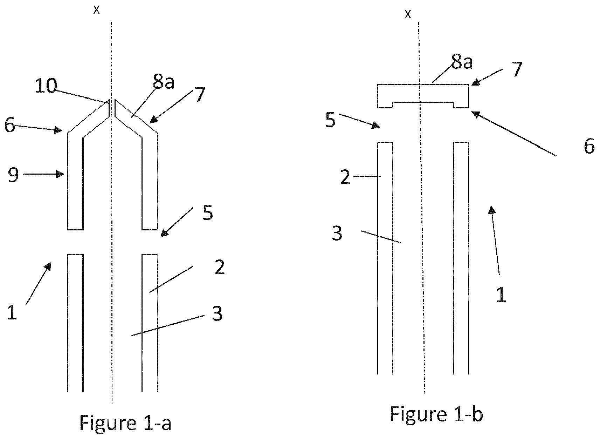

Les cathéters représentés aux figures 1b, 2b, 3 et 7a et décrits ci-dessous dans les passages correspondant à ces figures ne forment pas partie de l'invention mais décrivent des exemples utiles à la compréhension de l'invention.

DESCRIPTION DETAILLEE

La présente invention concerne un cathéter vasculaire 1 mono-lumière destiné à être inséré dans un canal de flux sanguin pour l'injection d'un produit par voie veineuse ou artérielle, comportant un tube 2 ayant un corps longitudinal creux suivant un axe longitudinal X présentant une lumière 3 (ou section transversale intérieure du tube 2) ici circulaire s'étendant tout le long du tube 2 et destinée à recevoir un volume 4 donné de produit, selon un débit prescrit.

Le tube 2 présente des orifices latéraux 5 qui sont disposés autour de l'axe longitudinal à proximité les uns des autres axialement selon l'axe longitudinal, les orifices latéraux 5 étant situés le long de l'axe longitudinal X avant une extrémité distale 6 et une surface d'extrémité 7.

L'expression 'à proximité' du paragraphe ci-dessus signifie un écart axial entre les orifices latéraux 5, inférieur à une dimension voisine d'un diamètre des orifices latéraux 5, voire de façon, avantageuse, nul ou proche de zéro.

Avantageusement, les orifices latéraux 5 sont sensiblement symétriques (de taille sensiblement identique et disposés sensiblement symétriquement par rapport à la lumière 3 dans le corps longitudinal), uniquement à proximité de l'extrémité distale 6 du tube 2.

La surface d'extrémité 7 réduit au moins partiellement la lumière 3 à l'extrémité distale 6 du tube 2.

La surface d'extrémité 7 est située après l'extrémité distale 6 et les orifices latéraux 5 sont situés avant l'extrémité distale 6, par rapport à la direction longitudinale X orientée de l'entrée du cathéter vers la sortie du cathéter qui permet d'éjecter le produit.

L'expression 'à proximité' du paragraphe ci-dessus signifie un positionnement des orifices latéraux 5 par rapport à l'extrémité distale 6 du tube 2 à des distances inférieures à celle d'un diamètre du tube 2.

Les orifices latéraux 5 sensiblement égaux et sensiblement symétriques et la surface d'extrémité 7 sont agencés et dimensionnés pour que le volume 4 sorte du cathéter par les orifices latéraux 5 sensiblement symétriques et entoure l'extrémité du cathéter, afin de stabiliser le tube 2 (limitant l'effet de fouet) et de diffuser le produit dans le canal de flux sanguin sur une grande surface radiale au tube 2.

La symétrie des orifices latéraux 5 permet que les forces de réaction provoquées par l'injection au niveau des orifices latéraux 5 soient symétriques (égales et opposées), et permet d'éviter la déportation du cathéter vers la paroi du vaisseau (risque d'endommagement de l'endothélium et/ou parois vasculaires), évitant tout effet de fouet, et limitant le retrait du cathéter.

Le cathéter limite les éventuels déplacements lors de rinçage pulsé.

L'adverbe 'sensiblement' pour la taille des orifices latéraux 5 qualifie le fait que les orifices latéraux peuvent être de taille identique à 20% (avantageusement de préférence à 10%, et encore plus avantageusement de préférence à 5%) près de leurs dimensions, le plus petit diamètre d'un orifice latéral n'étant pas inférieur à 80% du diamètre le plus grand d'un autre orifice latéral (avantageusement de préférence à 90%, et encore plus avantageusement de préférence à 95%).

L'adverbe 'sensiblement' pour le positionnement des orifices latéraux 5 qualifie le fait que les orifices latéraux 5 peuvent avoir un positionnement symétrique par rapport à l'axe longitudinal suivant la périphérie du tube à 20% près de leurs dimensions (par exemple du diamètre le plus grand), avantageusement de préférence à 10%, et encore plus avantageusement de préférence à 5%.

Il va de soi que l'effet de fouet (déplacement longitudinal) et l'effet de déplacement radial sont très réduits pour des orifices latéraux rigoureusement identiques en taille et qui ont un positionnement rigoureusement symétrique par rapport à l'axe longitudinal (la distribution des débits latéraux par les orifices latéraux étant identique ou quasi identique à ce moment-là), et sont plus grands pour des différences de diamètres ou de positionnement des orifices latéraux par rapport à l'axe longitudinal de l'ordre de 15 à 20%

Les figures 1-a et 1-b montrent respectivement un mode de réalisation de l'invention et un exemple ne formant pas partie de l'invention qui diffèrent l'un de 'autre.

Dans le premier mode de réalisation de la figure 1a, à l'extrémité du cathéter est profilée pour la surface d'extrémité 7 une forme dépassant de l'extrémité, percée d'un orifice central 10 de faible diamètre permettant uniquement le rinçage de l'extrémité du cathéter ou le passage d'un guide.

L'orifice central 10 a une taille fixe qui n'est pas destinée à se déformer élastiquement (augmenter de diamètre) lors du passage du bolus.

Le rapport diamètre orifice central 10/diamètre orifice latéral peut être par exemple de 10 à 15%.

La forme de la surface d'extrémité 7 a une diminution progressive de la taille de la lumière 3.

En l'occurrence ici, il s'agit d'une forme de cône 8a pour la surface d'extrémité 7 percée d'un trou de faible diamètre. Deux ouvertures latérales ou orifices latéraux 5 symétriques sont disposées à l'extrémité du cathéter (cf. figure 1-a, 1-b).

Ces ouvertures latérales symétriques permettent la sortie du fluide à injecter. Leur diamètre est prévu de manière à s'approcher au plus près de la résistance hydraulique qu'aurait le cathéter à extrémité ouverte à plein canal (dit à terminaison ouverte) non pourvu de ce dispositif.

L'orifice terminal 10 (cf. figure 1-a) a pour objet de permettre un flux de sortie favorisant un rinçage de l'extrémité du cathéter, sans risques de dépôts en utilisation à haut débit (produit de contraste) ou en injection thérapeutique à bas débit (antibiotique, cytolytique, alimentation parentérale...).

L'orifice terminal 10 permet en outre l'utilisation d'un guide (guidewire) favorisant le positionnement du cathéter selon la technique habituelle (Seldinger ou autres techniques similaires).

Par ailleurs, une partie longitudinale extérieure 9 distale du corps longitudinal est située entre les orifices latéraux 5 et la surface d'extrémité 7 du tube 2, et est entourée par le volume 4 de produit injecté hors du cathéter.

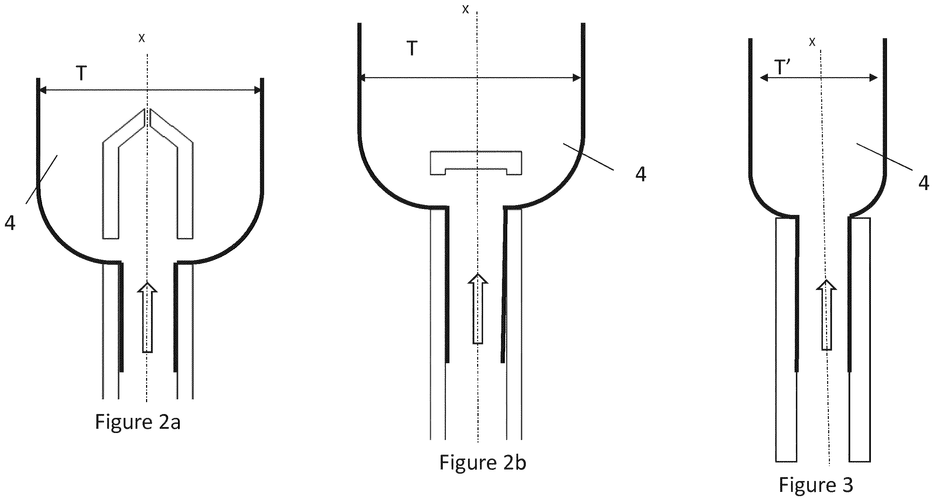

La longueur de la partie longitudinale extérieure 9 distale entre les orifices latéraux 5 et l'extrémité distale 6 du tube 2 peut être inférieure au rayon R du cathéter, à deux ou trois diamètres. A noter que les schémas de l'ensemble des figures et en particulier des figures 1a et 2a montrent le principe de l'invention mais ne sont pas nécessairement représentatifs de l'échelle de l'invention.

Avantageusement sans que cela soit limitatif, la surface d'extrémité 7 réduit au moins 90%, de préférence 95%, la surface totale de la lumière 3 à l'extrémité distale 6 du tube 2 adaptée à la taille du guide utilisé pour positionner le tube 2.

Il peut aussi être envisagé sans que cela soit limitatif que les orifices latéraux 5 et la surface d'extrémité 7 soient agencés et dimensionnés pour qu'au moins 90%, de préférence 95%, du volume 4 sorte du cathéter par les orifices latéraux 5 selon la taille du tube 2.

Dans le deuxième exemple ne formant pas partie de l'invention de la figure 1b, la surface d'extrémité 7 est une extrémité droite 8b et la surface d'extrémité 7 ferme complètement la lumière 3, le volume 4 étant injecté en sortie du cathéter à 100% par les orifices latéraux 5.

Ici, il est représenté que le tube 2 comporte deux orifices latéraux 5 en regard.

Dans d'autres réalisations, il pourrait être réalisé trois orifices disposés de façon symétrique par rapport à l'axe longitudinal (à 60° les uns des autres), ou 4 orifices avec deux paires d'orifices identiques en regard.

Les orifices latéraux 5 sont avantageusement réalisés transversalement (ou droits par rapport à la surface longitudinale du corps longitudinal suivant l'axe X) au corps longitudinal et sont ici sensiblement circulaires.

Les figures 2a, 2b et 3 montrent, à titre d'exemple, la formation du bolus ou volume 4 injecté avec une concentration donnée en sortie de cathéter pour les deux situations : figures 2a et 2b dans le cadre du cathéter à terminaison quasi-fermée selon l'invention ou fermée selon un exemple ne formant pas partie de l'invention. figure 3 dans le cadre d'un cathéter classique à terminaison ouverte selon un exemple ne formant pas partie de l'invention.

Ces schémas sont issus de deux simulations présentées dans les mêmes conditions avec une vitesse d'entrée dans le cathéter : 10cm/s, vitesse d'entrée dans la veine 1cm/s, diamètre de la veine 3cm, diamètre interne du cathéter 2mm. Ces simulations montrent une répartition uniforme de la concentration dans l'ensemble du volume 4 de produit sauf aux limites du volume 4 en contact avec le flux sanguin. Un gradient très fort est observé à ces limites.

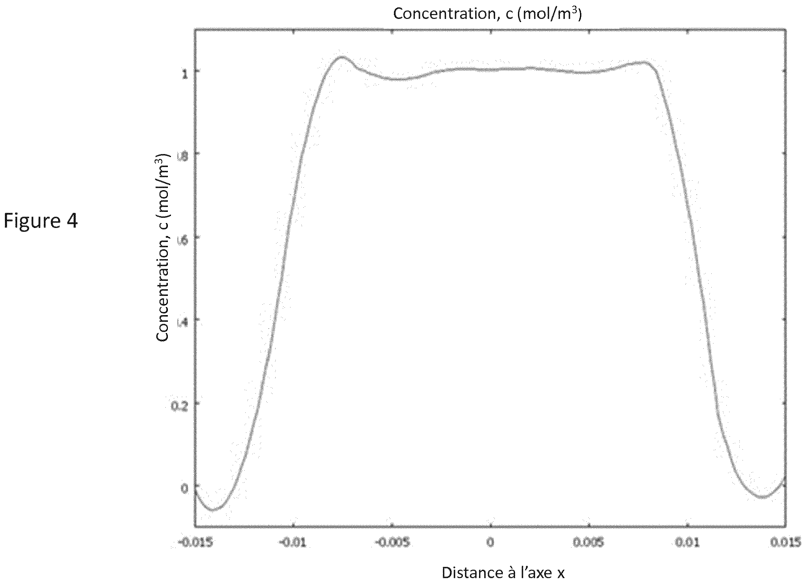

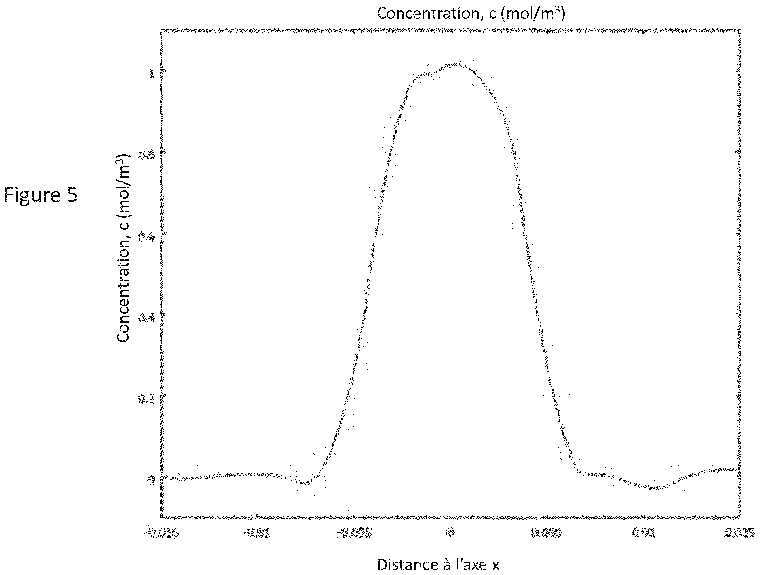

Les figures 4 et 5 montrent schématiquement des distributions de concentration en sortie de cathéter dans les deux cas présentés dans les figures 2a, 2b et 3. On constate à l'évidence sur la figure 4 que le profil de concentration est largement plus étalé (distances radiales T et T') dans la figure 4 correspondant à une injection latérale.

La distance radiale T correspond, à la sortie du cathéter (à une distance longitudinale de l'extrémité du cathéter égale à un diamètre du vaisseau sanguin), sensiblement au diamètre du vaisseau alors que la distance radiale T' à la sortie du cathéter (à une distance longitudinale de l'extrémité du cathéter égale à un diamètre du vaisseau sanguin) de l'état de l'art est seulement légèrement plus grande que le diamètre du cathéter. En d'autres termes, T est plus grande ou bien plus grande que T'.

La distance T'va augmenter lorsque l'on s'éloigne du cathéter dans le sens du flux, mais la dilution du produit va être plus importante qu'en sortie de cathéter, contrairement à l'invention où tout de suite à la sortie du cathéter, le bolus concentré de la taille du diamètre du canal est disponible. Ce qui permet une grande qualité d'image des vaisseaux ciblés, le bolus étant bien réparti radialement suivant T et peu longitudinalement suivant l'axe X (contrairement à l'état de l'art de la figure 3 où le bolus est mieux réparti longitudinalement suivant l'axe X que radialement suivant T'). Cette distance T' est dépendante pour un temps d'éjection donné, de la distance parcourue par le fluide sanguin.

Cet avantage permet ainsi de mettre moins de produit de contraste que dans l'état de l'art de la figure 3, néfaste pour la santé, au moins à qualité comparable avec ce qui est obtenu avec l'état de l'art de la figure 3. Ou autrement dit, une qualité d'image égale à toxicité moindre ou une meilleure qualité d'image à quantité égale de produit de contraste.

En outre, et comme déjà décrit, le cathéter selon l'invention permet d'éviter l'effet de fouet et ne présente pas d'effet de recul et / ou de mobilisation latérale en fonctionnement.

Les orifices latéraux 5 présentent une dimension longitudinale suivant l'axe X calculée pour que la concentration du volume 4 de produit en sortie de cathéter tende à être homogène sur une distance radiale T, par rapport et perpendiculaire à un axe longitudinal X central du cathéter et à proximité de l'extrémité du cathéter, égale à au moins quatre fois le rayon R.

La résistance hydraulique est le paramètre pertinent permettant de caractériser les performances hydrodynamiques d'un cathéter. Rappelons que la résistance hydraulique est définie, en situation d'écoulement

par $Rh = \dfrac{\nabla P}{Q}$, où Q est le débit de perfusion dans un conduit de longueur donnée avec la différence de pression VP appliquée entre les extrémités distale et proximale du conduit.

A titre d'exemple, on a conduit différentes simulations numériques afin d'estimer l'impact d'orifices latéraux 5 symétriques sur la résistance hydraulique d'un cathéter.

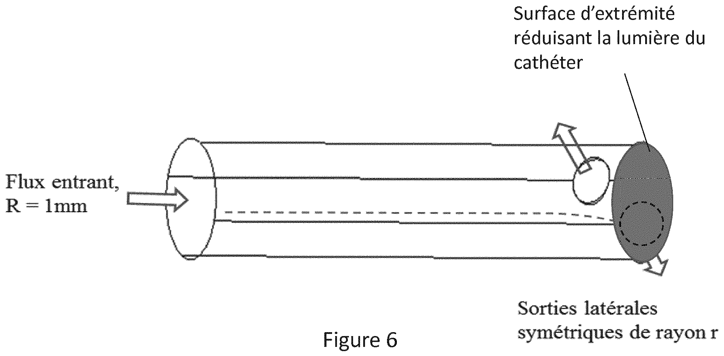

La figure 6 montre les notations retenues : R désigne le rayon du cathéter et r désigne le rayon des ouvertures latérales symétriques et correspond à une dimension longitudinale caractéristique des ouvertures latérales. On a choisi et la résistance hydraulique sera calculée pour différentes valeurs de r.

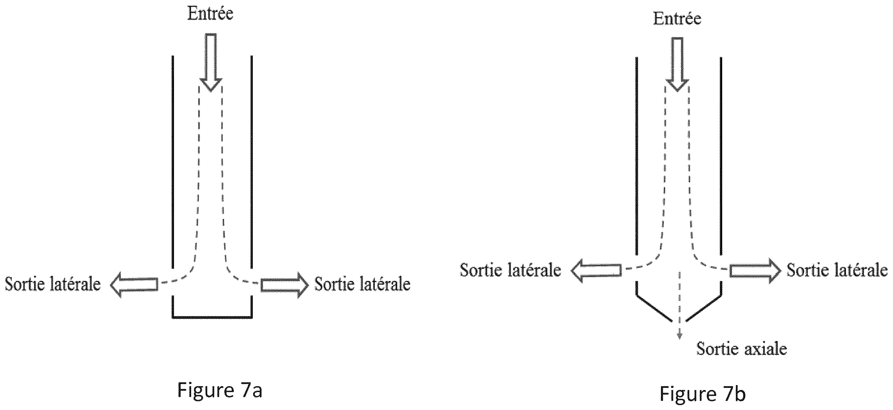

Les figures 7a et 7b rappellent la géométrie de l'extrémité du cathéter ainsi que les lignes d'écoulement suivant que l'on retient deux ouvertures latérales symétriques (Figure 7a) selon un exemple ne formant pas partie de l'invention ou deux ouvertures latérales symétriques complétées par une petite ouverture axiale permettant un meilleur rinçage (Figure 7b) selon l' invention.

On a calculé la résistance hydraulique $R_h$ d'un segment du cathéter pour différentes valeurs de r et on a comparé ce résultat à la résistance hydraulique $R_{h0}$ du même segment comportant un seul orifice distal de sortie (à viscosité égale) dit à terminaison ouverte.

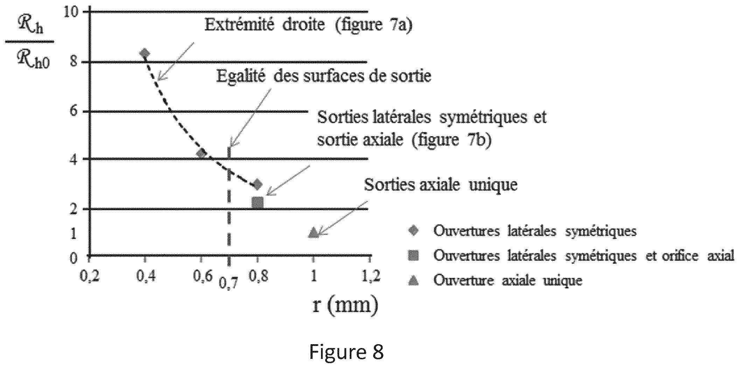

La figure 8 montre les résultats obtenus. Il est évident que le remplacement d'une ouverture distale axiale par deux ouvertures latérales symétriques conduit à augmenter la résistance hydraulique même si la surface totale des ouvertures latérales symétriques est identique à la surface axiale de sortie (ligne pointillée, sur la figure 8).

Précisons que l'égalité des surfaces est évidemment obtenue pour $r = \dfrac{R}{\sqrt{2}}$ où R désigne le rayon endoluminal du cathéter et r le rayon de chaque orifice latéral.

Cette augmentation de résistance est évidemment due à l'introduction d'une courbure des lignes d'écoulement (cf. lignes pointillées dans les figures 7a et 7b).

On constate que cette augmentation est moindre si on ménage une légère ouverture à l'extrémité distale (cf. figure 7b). Les résultats sont présentés en comparant les résistances hydrauliques d'un cathéter ouvert à l'extrémité à la résistance hydraulique d'un cathéter comportant des orifices latéraux 5 symétriques. Cette comparaison est évidemment faite pour un même fluide injecté. En d'autres termes ces résultats sont peu dépendants de la viscosité du fluide perfusé et sont donc représentatif de la géométrie du cathéter, ce qui confirme l'intérêt du dispositif proposé.

En d'autres termes, la lumière 3 présentant une surface totale donnée et un rayon R donné, les orifices latéraux 5 peuvent présenter une dimension longitudinale suivant l'axe X calculée pour qu'une somme des surfaces individuelles des orifices latéraux 5 soit au moins égale à la surface totale donnée de la lumière 3.

La longueur de la partie extérieure 9 distale entre les orifices latéraux 5 et l'extrémité distale 6 du tube 2 peut être choisie pour être est la plus faible possible, mécaniquement supportable pour le matériau du cathéter et le procédé de fabrication employé.

Une distance comprise entre un rayon et un diamètre du tube 2 devrait être raisonnable pour assurer une possibilité de rinçage satisfaisante et une résistance mécanique correcte, mais cet exemple n'est pas limitatif de l'invention.

L'autre extrémité, proximale, du cathéter est apte à être raccordée à un embout connectable type luer.

Les cathéters peuvent être installés de façon prolongée ou provisoire dans le corps humain.

La pose d'un cathéter vasculaire 1 permet de réaliser quatre objectifs d'indication diverse :

-   prise de pression intravasculaire artérielle et/ou veineuse permettant d'évaluer et de surveiller l'hémodynamique cardiovasculaire.
-   prélèvements sanguins aux fins d'analyses biologiques
-   administration continue ou séquentielle de thérapeutiques veineuses ou artérielles
-   administration isolée et/ou itérative de produits de contraste réalisant une opacification vasculaire (radiologie ou cardiologie interventionnelles).

Le cathéter vasculaire 1 prescrit selon l'invention est posé pour une durée variable : ponctuelle ou prolongée.

Jusqu'à présent, chaque type d'objectif édictait la pose d'un type cathéter spécifique.

Actuellement, bien que parfois controversée, l'utilisation, pour un examen ponctuel (exploration vasculaire), d'un cathéter veineux de longue ou très longue durée est préconisée pour réduire l'atteinte corporelle (lésion du réseau vasculaire) et favoriser l'examen radiologique (réduction de volume et meilleure concentration du produit injecté).

L'invention peut être réalisée et utilisée pour tout type de cathéter :

- de cardiologie ou radiologie interventionnelle d'utilisation ponctuelle artérielle ou veineuse, sous faible ou haute pression ou débit.
- d'administration thérapeutique (Midline, PICCline, PICC Port ou Cathéter à chambre implantable qualifiés ou non Haute Pression) de longue ou très longue durée, et utilisable pour toute injection à moindre débit.

En ce sens, le cathéter selon l'invention est un cathéter multi applications utilisable pour une grande échelle de pressions différentes et de débit différent (par exemple à titre uniquement illustratif pour des applications avec des pressions de l'ordre de 551,58 à 2413,17 KPa (80 à 350 psi) et des débits infimes ou extrêmes.

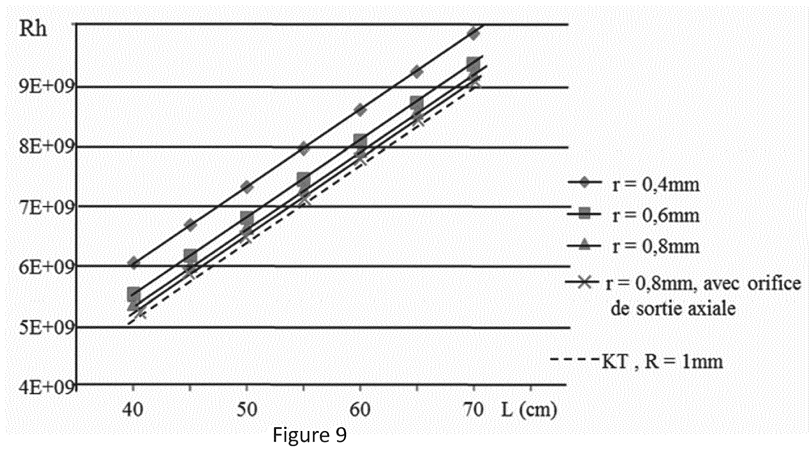

On peut maintenant représenter la résistance hydraulique totale Rh d'un cathéter de longueur L comportant deux orifices latéraux 5 symétriques de sortie. Les résultats sont représentés dans la figure 9 pour différentes valeurs du rayon r des orifices latéraux 5 pour une longueur de cathéter égale à 70cm.

On a représenté en pointillé la résistance hydraulique totale du cathéter ne comportant qu'un seul orifice distal de sortie. On voit donc qu'en donnant aux orifices de sortie une surface identique à la surface de la lumière 3 du cathéter, on ne modifie qu'à la marge la résistance hydraulique totale du cathéter (pour l'injection d'un produit de même viscosité).

Pour un cathéter de longueur égale, la variation de résistance hydraulique totale n'excède pas 3% (pour une même viscosité), ce qui est négligeable dans une utilisation pour injection de produits de contraste.

En d'autres termes, les orifices latéraux 5 présentent une dimension longitudinale calculée pour que, à longueur égale du cathéter, la différence entre une résistance hydraulique totale calculée du cathéter selon l'invention, par rapport à une résistance hydraulique totale de référence calculée d'un cathéter à terminaison ouverte destiné à s'ouvrir à plein canal et ayant à l'extrémité un orifice longitudinal de sortie correspondant à la lumière 3, peut être inférieure à 5% de la résistance hydraulique totale de référence.

En résumé, on peut estimer au vu des résultats précédents, que le meilleur choix pour le rayon r des ouvertures latérales symétriques se situe dans le voisinage l'intervalle : $r \approx \dfrac{R}{\sqrt{2}}$

La présence d'une ouverture distale de faible rayon est utile pour assurer un meilleur rinçage en particulier si le cathéter est destiné à rester en place (cathéter central, PICC Port et PICC line) mais aussi pour permettre le passage du guideline adapté. Le diamètre de cette ouverture distale est variable selon le diamètre du cathéter et du leader utilisé (18-22G).

Cette ouverture centrale distale 10 permet seulement de laisser passer un filet de produit pour le rinçage de l'extrémité du cathéter, et il est choisi des guides de petit diamètre par rapport aux orifices latéraux de rayon r du tube.

Le rapport entre le diamètre des orifices latéraux et le diamètre de l'orifice central 10 est supérieur ou égal à deux, avantageusement supérieur ou égal à quatre.

Il est d'abord choisi un guide de diamètre d, puis réalisé l'orifice central 10 et les orifices latéraux 5 dans le cathéter, pour que l'écoulement du produit sorte essentiellement par les orifices latéraux et de façon négligeable par l'orifice central 10 (moins de 10% du volume, avantageusement moins de 5%).

Tous les cathéters vasculaires mono lumières de toutes longueurs et tous diamètres sont concernés par cette proposition.

Par exemple, ils peuvent présenter un diamètre de 3French à 12French (0,9 à 4mm).

Par exemple, ils peuvent avoir toute longueur, des longueurs supérieures à 50 cm.

**Revendications**

1. Cathéter vasculaire (1) mono-lumière artériel ou veineux destiné à être inséré dans un canal de flux sanguin permettant l'injection d'un produit en bolus sous forme de fluide par voie veineuse ou artérielle, comportant un tube (2) ayant un corps longitudinal creux ayant un axe longitudinal (X), une longueur donnée et présentant une lumière (3) avec une surface totale donnée et un rayon R donné, la lumière (3) s'étendant tout le long du tube (2) et étant destinée à permettre l'injection d'un volume (4) donné de produit, selon un débit prescrit,
le tube (2) présentant des orifices latéraux (5) symétriques permettant la sortie du volume (4) radialement au tube (2), de taille identique, disposés symétriquement par rapport à l'axe longitudinal (X) dans le corps longitudinal, uniquement à proximité et avant une extrémité distale (6) du tube (2) selon la direction de l'axe longitudinal (X), et **caractérisé en ce que** :

- une surface d'extrémité (7) située à partir de l'extrémité distale (6) du tube (2) selon la direction de l'axe longitudinal (X), réduit une surface totale de la lumière (3) à partir de l'extrémité distale (6) du tube (2), à une forme de tronc de cône endoluminal (8a) percée d'un orifice central (10),
- l'orifice central (10) étant configuré pour recevoir un guide, l'orifice central (10) étant de la taille du guide et servant uniquement : à placer le guide et à permettre le rinçage de l'extrémité distale du tube, le cathéter vasculaire étant dit à terminaison quasi-fermée ;
- l'orifice central (10) ayant une taille fixe qui n'est pas destinée à se déformer élastiquement lors du passage du bolus,
- les orifices latéraux (5) du cathéter présentent une dimension calculée pour que, selon un débit prescrit d'un même fluide, la différence entre :

    - une résistance hydraulique totale calculée sur toute la longueur donnée du cathéter, et
    - une résistance hydraulique totale de référence calculée avec un cathéter de référence à terminaison ouverte destinée à s'ouvrir à plein canal, ayant à l'extrémité uniquement un orifice longitudinal de sortie du fluide correspondant à la lumière (3), et une longueur égale à la longueur donnée,

soit inférieure à 5% de la résistance hydraulique totale de référence,

    - les orifices latéraux (5) présentent une dimension longitudinale suivant l'axe X, calculée pour qu'une somme des surfaces individuelles des orifices latéraux (5) soit au moins égale à la surface totale donnée de la lumière (3),
    - les orifices latéraux (5) et la surface d'extrémité (7) sont agencés et dimensionnés pour qu'au moins 90% du volume (4) sorte du cathéter par les orifices latéraux (5) selon un débit prescrit de fluide.

2. Cathéter vasculaire (1) mono-lumière selon la revendication 1, **caractérisé en ce que** les orifices latéraux (5) présentent une dimension longitudinale suivant l'axe X calculée pour que la concentration du volume (4) de produit en sortie de cathéter, soit homogène sur une distance radiale T, par rapport et perpendiculaire à l'axe longitudinal X central du cathéter et à proximité de l'extrémité du cathéter, égale à au moins quatre fois le rayon R.

3. Cathéter vasculaire (1) mono-lumière selon l'une des revendications 1 à 2, **caractérisé en ce que** la longueur d'une partie longitudinale extérieure (9) distale située entre les orifices latéraux (5) et l'extrémité distale (6) du tube (2) est inférieure à trois diamètres du cathéter.

4. Cathéter vasculaire (1) mono-lumière selon l'une des revendications 1 à 3, **caractérisé en ce que** la longueur de la partie extérieure (9) distale entre les orifices latéraux (5) et l'extrémité distale (6) du tube (2) est choisie pour être est la plus faible possible, mécaniquement supportable pour le matériau du cathéter et le procédé de fabrication employé.

5. Cathéter vasculaire (1) mono-lumière selon l'une des revendications 1 à 4, **caractérisé en ce que** les orifices latéraux (5) sont réalisés droits par rapport à la surface longitudinale du corps longitudinal du tube (2) suivant l'axe X.

6. Cathéter vasculaire (1) mono-lumière selon l'une des revendications 1 à 5, **caractérisé en ce que** les orifices latéraux (5) sont situés à une seule position suivant l'axe longitudinal (X).

7. Cathéter vasculaire (1) mono-lumière selon l'une des revendications 1 à 6, **caractérisé en ce que** la surface d'extrémité (7) réduit d'au moins 90% la surface totale donnée de la lumière (3) considérée à l'extrémité distale (6).

8. Cathéter vasculaire (1) mono-lumière selon l'une des revendications 1 à 7, **caractérisé en ce que** le rapport entre le diamètre des orifices latéraux et le diamètre de l'orifice central est supérieur ou égal à deux.

9. Cathéter vasculaire (1) mono-lumière selon l'une des revendications 1 à 8, **caractérisé en ce que** le rapport entre le diamètre des orifices latéraux et le diamètre de l'orifice central est supérieur ou égal à quatre.

10. Cathéter vasculaire (1) mono-lumière selon l'une des revendications 1 à 9, **caractérisé en ce que** le rapport diamètre orifice central/diamètre orifice latéral est de 10 à 15%.

11. Cathéter vasculaire (1) mono-lumière selon l'une des revendications 1 à 10, **caractérisé en ce que** les orifices latéraux (5) sont dimensionnés pour faire sortir des débits d'injection de bolus de 0,5 à 5 mL/s, à des pressions de l'ordre de 551,58 à 2413,17 KPa (80 à 350 psi).

12. Cathéter vasculaire (1) mono-lumière selon l'une des revendications 1 à 11, **caractérisé en ce que** les orifices latéraux (5) présentent un rayon r calculé, pour qu'une somme des surfaces individuelles des orifices latéraux (5) soit au moins égale à la surface totale donnée de la lumière (3) considérée à l'extrémité distale (6), à savoir le

R rayon r est au moins égal à : $r = \dfrac{R}{\sqrt{2}}$.

**13.** Cathéter vasculaire (1) mono-lumière selon l'une des revendications 1 à 12, **caractérisé en ce que** le tube (2) comporte deux orifices latéraux (5) en regard.

**14.** Cathéter vasculaire (1) mono-lumière selon l'une des revendications 1 à 12, **caractérisé en ce que** le tube (2) comporte trois orifices latéraux (5).

**15.** Cathéter vasculaire (1) mono-lumière selon l'une des revendications 1 à 12, **caractérisé en ce que** le tube (2) comporte 4 orifices, avec deux paires d'orifices identiques en regard.

**Patentansprüche**

**1.** Arterieller oder venöser Gefäßkatheter (1) mit einer Öffnung, der zum Einfügen in einen Blutstromkanal bestimmt ist und das Einspritzen eines Produkts als Bolus in Form einer Flüssigkeit auf venösem oder arteriellem Weg erlaubt, umfassend eine Röhre (2) mit einem hohlen Längskörper mit einer Längsachse (X), einer bestimmten Länge und der eine Öffnung (3) mit einer bestimmten Gesamtoberfläche und einem bestimmten Radius R aufweist, wobei sich die Öffnung (3) über die gesamte Länge der Röhre (2) erstreckt und dazu bestimmt ist, das Einspritzen eines bestimmten Volumens (4) des Produkts gemäß einem vorgeschriebenen Durchsatz zu erlauben, wobei die Röhre (2) seitliche symmetrische Löcher (5) aufweist, die den Ausgang des Volumens (4) radial zur Röhre (2) mit identischer Größe erlauben, die symmetrisch im Verhältnis zur Längsachse (X) in dem Längskörper, ausschließlich in der Nähe und vor dem distalen Ende (6) der Röhre (2) gemäß der Richtung der Längsachse (X) angeordnet sind, und **dadurch gekennzeichnet, dass**:

- eine Endoberfläche (7), die ausgehend von dem distalen Ende (6) der Röhre (2) gemäß der Richtung der Längsachse (X) angeordnet ist, eine Gesamtoberfläche der Öffnung (3) ausgehend von dem distalen Ende (6) der Röhre (2) zu einer endoluminalen Kegelstumpfform (8a) verringert, die mit einem zentralen Loch (10) durchbohrt ist,
- zentrales Loch (10), das ausgestaltet ist, um eine Führung aufzunehmen, wobei das zentrale Loch (10) die Größe der Führung aufweist und ausschließlich dient: zum Platzieren der Führung und zum Erlauben der Spülung des distalen Endes der Röhre, wobei der Gefäßkatheter mit einer so genannten quasi geschlossenen Endung versehen ist;
- zentrales Loch (10) mit einer festen Größe, die nicht dazu bestimmt ist, sich beim Durchgang des Bolus elastisch zu verformen,
- die seitlichen Öffnungen (5) des Katheters weisen eine Abmessung auf, die berechnet ist, damit der Unterschied gemäß einem vorgeschriebenen Durchsatz einer und derselben Flüssigkeit zwischen:

    - einem hydraulischen Gesamtwiderstand, der über die gesamte bestimmte Länge des Katheters berechnet ist und
    - einem hydraulischen Referenzgesamtwiderstand, der mit einem Referenzkatheter mit offener Endung berechnet ist, die dazu bestimmt ist, sich direkt auf den Kanal zu öffnen und am Ende nur ein längliches Ausgangsloch der Flüssigkeit, das der Öffnung (3) entspricht, und eine Länge gleich der bestimmten Länge aufweist, unter 5 % des hydraulischen Referenzgesamtwiderstandes liegt,

- die seitlichen Löcher (5) eine Längsabmessung gemäß der Achse X aufweisen, die berechnet ist, damit eine Summe der einzelnen Oberflächen der seitlichen Löcher (5) wenigstens gleich der bestimmten Gesamtoberfläche der Öffnung (3) ist,
- die seitlichen Löcher (5) und die Endoberfläche (7) angeordnet und bemessen sind, damit wenigstens 90 % des Volumens (4) aus dem Katheter durch die seitlichen Löcher (5) gemäß einem vorgeschriebenen Durchsatz der Flüssigkeit austritt.

**2.** Gefäßkatheter (1) mit einer Öffnung gemäß Anspruch 1, **dadurch gekennzeichnet, dass** die seitlichen Löcher (5) eine Längsabmessung gemäß der Achse X aufweisen und die berechnet ist, damit die Konzentration des Volumens (4) des Produkts am Ausgang des Katheters über eine radiale Entfernung T im Verhältnis und lotrecht zur zentralen Längsachse X des Katheters und in der Nähe des Endes des Katheters homogen, gleich wenigstens des Vierfachen des Radius R ist.

**3.** Gefäßkatheter (1) mit einer Öffnung gemäß Anspruch 1 bis 2, **dadurch gekennzeichnet, dass** die Länge eines äußeren distalen Längsteils (9), die zwischen den seitlichen Löchern (5) und dem distalen Ende (6) der Röhre angeordnet ist, kleiner ist als drei Durchmesser des Katheters.

**4.** Gefäßkatheter (1) mit einer Öffnung gemäß Anspruch 1 bis 3, **dadurch gekennzeichnet, dass** die Länge des distalen äußeren Teils (9) zwischen den seitlichen Löchern (5) des distalen Endes (6) der Röhre gewählt ist, um so kurz wie möglich, mechanisch für das Material des Katheters und das verwendete Herstellungsverfahren tragbar zu sein.

5. Gefäßkatheter (1) mit einer Öffnung gemäß Anspruch 1 bis 4, **dadurch gekennzeichnet, dass** die seitlichen Löcher (5) im Verhältnis zur Längsoberfläche des länglichen Körpers der Röhre (2) gemäß der Achse X ausgeführt sind.

6. Gefäßkatheter (1) mit einer Öffnung gemäß Anspruch 1 bis 5, **dadurch gekennzeichnet, dass** die seitlichen Löcher (5) in einer einzigen Position gemäß der Längsachse (X) angeordnet sind.

7. Gefäßkatheter (1) mit einer Öffnung gemäß Anspruch 1 bis 6, **dadurch gekennzeichnet, dass** die Endoberfläche (7) die bestimmte Gesamtoberfläche der betrachteten Öffnung (3) am distalen Ende (6) um wenigstens 90 % verringert.

8. Gefäßkatheter (1) mit einer Öffnung gemäß Anspruch 1 bis 7, **dadurch gekennzeichnet, dass** das Verhältnis zwischen dem Durchmesser der seitlichen Löcher und dem Durchmesser des zentralen Lochs größer als oder gleich zwei ist.

9. Gefäßkatheter (1) mit einer Öffnung gemäß Anspruch 1 bis 8, **dadurch gekennzeichnet, dass** das Verhältnis zwischen dem Durchmesser der seitlichen Löcher und dem Durchmesser des zentralen Lochs größer als oder gleich vier ist.

10. Gefäßkatheter (1) mit einer Öffnung gemäß einem der Ansprüche 1 bis 9, **dadurch gekennzeichnet, dass** das Verhältnis Durchmesser zentrales Loch / Durchmesser seitliches Loch 10 bis 15 % beträgt.

11. Gefäßkatheter (1) mit einer Öffnung gemäß einem der Ansprüche 1 bis 10, **dadurch gekennzeichnet, dass** die seitlichen Löcher (5) bemessen sind, um Einspritzdurchsätze des Bolus von 0,5 bis 5 ml/Sek. bei Drücken in der Größenordnung von 551,58 bis 2413,17 KPa (80 bis 350 psi) austreten zu lassen.

12. Gefäßkatheter (1) mit einer Öffnung gemäß einem der Ansprüche 1 bis 11, **dadurch gekennzeichnet, dass** die seitlichen Löcher (5) einen berechneten Radius r aufweisen, damit eine Summe der einzelnen Oberflächen der seitlichen Löcher (5) wenigstens gleich der betrachteten bestimmten Gesamtoberfläche (3) am distalen Ende (6) ist, und zwar ist der Radius r $r = \dfrac{R}{\sqrt{2}}$ wenigstens gleich:

13. Gefäßkatheter (1) mit einer Öffnung gemäß einem der Ansprüche 1 bis 12, **dadurch gekennzeichnet, dass** die Röhre (2) zwei seitliche gegenüberliegende Löcher (5) umfasst.

14. Gefäßkatheter (1) mit einer Öffnung gemäß einem der Ansprüche 1 bis 12, **dadurch gekennzeichnet, dass** die Röhre (2) drei seitliche Löcher (5) umfasst.

15. Gefäßkatheter (1) mit einer Öffnung gemäß einem der Ansprüche 1 bis 12, **dadurch gekennzeichnet, dass** die Röhre (2) 4 Löcher mit zwei identischen gegenüberliegenden Lochpaaren aufweist.

**Claims**

1. An arterial or venous single-lumen vascular catheter (1) for being inserted into a blood flow channel for injecting a bolus product as a fluid by venous or arterial pathway, including a tube (2) with a hollow longitudinal body with a longitudinal axis (X), a given length and having a lumen (3) with a given total surface area and a given radius R, the lumen (3) extending all along the tube (2) and for enabling the injection of a product given volume (4), according to a prescribed flow rate,
the tube (2) having symmetric lateral ports (5) enabling exit of the volume (4) radially to the tube (2), of an identical size, which are symmetrically disposed with respect to the longitudinal axis (X) in the longitudinal body, only in proximity to and before a distal end (6) of the tube (2) along the direction of the longitudinal axis (X), and **characterised in that**:

- an end surface (7) located from the distal end (6) of the tube (2) along the direction of the longitudinal axis (X), reduces a total surface area of the lumen (3) from the distal end (6) of the tube (2), to an endoluminal frustoconical shape (8a) pierced with a central port (10),
- the central port (10) being configured to receive a guide, the central port (10) being of the size of the guide and being only used to: place the guide and enable the distal end of the tube to be rinsed, the vascular catheter being referred to as a nearly closed-end catheter;
- the central port (10) having a fixed size which is not to be elastically deformed during the passage of the bolus,
- the side ports (5) of the catheter have a dimension calculated so that, according to a prescribed flow rate of a same fluid, the difference between:
- a total hydraulic resistance calculated over the whole given length of the catheter, and
- a reference total hydraulic resistance calculated with a reference catheter with an open end for being open when the channel is full, having at the end only a fluid outlet longitudinal port corresponding to the lumen (3), and a length equal to the given length,
is lower than 5% of the reference total hydraulic resistance,
- the lateral ports (5) have a longitudinal dimension along the axis X, calculated so that a sum of the individual surface areas of the lateral ports (5) is at least equal to the given total surface area of the lumen (3),
- the lateral ports (5) and the end surface area (7) are arranged and sized so that at least 90% of the volume (4) exits from the catheter through the lateral ports (5) according to a fluid prescribed flow rate.

2. The single-lumen vascular catheter (1) according to claim 1, **characterised in that** the lateral ports (5) have a longitudinal dimension along axis X calculated so that a concentration of the product volume (4) at the catheter outlet, is homogenous over a radial distance T, with respect and perpendicular to the central longitudinal axis X of the catheter and in proximity to the catheter end, equal to at least four times the radius R.

3. The single-lumen vascular catheter (1) according to one of claims 1 to 2, **characterised in that** the length of a distal outer longitudinal part (9) located between the lateral ports (5) and the distal end (6) of the tube (2) is lower than three diameters of the catheter.

4. The single-lumen vascular catheter (1) according to one of claims 1 to 3, **characterised in that** the length of the distal outer part (9) between the lateral ports (5) and the distal end (6) of the tube (2) is selected to be the smallest possible, mechanically withstandable for the catheter material and the manufacturing method employed.

5. The single-lumen vascular catheter (1) according to one of claims 1 to 4, **characterised in that** the lateral ports (5) are made straight with respect to the longitudinal surface of the longitudinal body of the tube (2) along axis X.

6. The single-lumen vascular catheter (1) according to one of claims 1 to 5, **characterised in that** the lateral ports (5) are located in a single position along longitudinal axis (X).

7. The single-lumen vascular catheter (1) according to one of claims 1 to 6, **characterised in that** the end surface (7) reduces by at least 90% the given total surface area of the lumen (3) considered at the distal end (6).

8. The single-lumen vascular catheter (1) according to one of claims 1 to 7, **characterised in that** the ratio of the diameter of the lateral ports to the diameter of the central port is greater than or equal to two.

9. The single-lumen vascular catheter (1) according to one of claims 1 to 8, **characterised in that** the ratio of the diameter of the lateral ports to the diameter of the central port is greater than or equal to four.

10. The single-lumen vascular catheter (1) according to one of claims 1 to 9, **characterised in that** the central port diameter/lateral port diameter ratio is 10 to 15%.

11. The single-lumen vascular catheter (1) according to one of claims 1 to 10, **characterised in that** the lateral ports (5) are sized to discharge flow rates of bolus injection of 0.5 to 5mL/s, at pressures in the order of 551.58 to 2413.17 KPa (80 to 350 psi).

12. The single-lumen vascular catheter (1) according to one of claims 1 to 11, **characterised in that** the lateral ports (5) have a radius r calculated, so that a sum of the individual surface areas of the lateral ports (5) is at least equal to the given total surface area of the lumen (3) considered at the distal end (6), namely the radius r is at least equal to: $r = \frac{R}{\sqrt{2}}$.

13. The single-lumen vascular catheter (1) according to one of claims 1 to 12, **characterised in that** the tube (2) includes two lateral ports (5) facing each other.

14. The single-lumen vascular catheter (1) according to one of claims 1 to 12, **characterised in that** the tube (2) includes three lateral ports (5).

15. The single-lumen vascular catheter (1) according to one of claims 1 to 12, **characterised in that** the tube (2) includes 4 ports, with two identical pairs of ports facing each other.

Figure 1-a

Figure 1-b

EP 3 297 716 B1

Figure 3

Figure 2b

Figure 2a

Figure 4

Figure 5

Entrée

Sortie latérale ⇐ ⇒ Sortie latérale

Figure 7a

Entrée

Sortie latérale ⇐ ⇒ Sortie latérale

Sortie axiale

Figure 7b

Figure 8

Figure 9

Surface d'extrémité
réduisant la lumière du
cathéter

Flux entrant,
R = 1mm

Sorties latérales
symétriques de rayon r

Figure 6